# EUROPEAN PATENT SPECIFICATION

(11) **EP 0 644 752 B1**
(45) Date of publication and mention of the grant of the patent: **19.12.2001**
(21) Application number: 93916550.2
(22) Date of filing: 15.06.1993
(51) Int. Cl.: A61K 7/48, A61K 35/78

(54) **COMPOSITION FOR THE TREATMENT OF SKIN**
ZUSAMMENSETZUNG ZUR HAUTBEHANDLUNG
COMPOSITION POUR LE TRAITEMENT DE LA PEAU

(30) Priority: 16.06.1992 US 899418; 24.05.1993 US 64915; 14.06.1993 US 76017
(43) Date of publication of application: 29.03.1995
(73) Proprietor: KLEIN, Marvin, Farmington Hills, MI 48334 (US)
(72) Inventor: KLEIN, Marvin, Farmington Hills, MI 48334 (US)
(74) Representative: MacDougall, Donald Carmichael
(86) International application number: US9305739
(87) International publication number: WO9325186

(56) References cited:
- GB-A- 2 232 587
- US-A- 3 057 467
- US-A- 4 234 599
- DATABASE WPI Week 9351 Derwent Publications Ltd., London, GB; AN 93-411978 XP002016595 & SU 1 779 373 A (RIGA DZINTARS PRODN ASSOC)
- STN, File Supplier, Karlsruhe, DE, File XP002016594
- DATABASE WPI Week 8704 Derwent Publications Ltd., London, GB; AN 87-028739 XP002016596 & SU 1 237 210 A (SOYUZBYTKHIM)
- DATABASE WPI Week 9350 Derwent Publications Ltd., London, GB; AN 93-400348 XP002016597 & JP 05 301 821 A (KAO CORP)

## Description

### Field of the Invention

This invention relates to dermatologic compositions in general. Specifically, the invention relates to dermatologic compositions which include an acidic material and a limonene based oil. The invention also relates to a packaged delivery system for acidic dermatologic compositions.

### Background of the Invention

Certain organic acids have an extensive history of use in connection with the treatment of skin conditions. Sour milk contains lactic acid and a variety of fruit juices contain glycolic acid. Both of these acids are alphahydroxy carboxylic acids and a variety of compositions based upon fruit juice or soured milk have long been documented as being beneficial for the skin. Similarly, polycarboxylic acids such as citric acid, which is present in many fruits and tartaric acid, which is present in wine residues, have also long been used as skin treatments. More recently, halo-carboxylic acids, primarily trichloracetic acid, have been found to be beneficial as skin treatment agents. These acidic materials, when properly applied, have been found to soften, moisturize and tone the skin, bleach unwanted pigmentation, reduce wrinkles and ameliorate conditions such as pseudofolliculitis barbae, keratoses.

A variety of compositions incorporating the aforementioned acidic materials have been known for some time in the prior art, and U.S. Patent Nos. 3,897,537; 3,984,566; 3,988,470 are representative of compositions including such acidic materials. However, it has come to be found that use of the free acids can actually irritate the skin, producing an uncomfortable burning sensation, red spots, and temporary hyperpigmentation. Furthermore, the free acids tend to crystallize out of various preparations thereby limiting their shelf life and further exacerbating the problem of irritation.

In response to problems associated with the use of free acids, the prior art turned to compositions including neutralized acids therein. The neutralization is typically accomplished by the use of inorganic bases such as sodium hydroxide, potassium hydroxide or ammonium hydroxide, which react with the acids to form salts. U.S. Patent Nos. 4,021,572; 4,105,783; and 4,234,599 are representative of the prior art relating to neutralized compositions. In general, it has been found that neutralization does prevent the irritating effects encountered in the use of acid containing preparations; however, it has also been found that the neutralized products are ineffective insofar as they do not rejuvenate, tone or bleach the skin. U.S. Patent No. 5,091,171 contains a discussion of the prior art compositions and acknowledges the inactivity of the salts resultant from the neutralization of the acids. The '171 patent proposes a skin treating composition comprising acids in conjunction with amphoteric materials such as amino acids, peptides. It has been found that all of the prior art approaches have been unsuccessful in providing an acid-based skin treatment composition which is both nonirritating and effective.

In accord with one aspect of the present invention, it has been found that the addition of even relatively small amounts of a particular group of oils, referred to as limonene oils eliminates problems heretofore encountered in the use of acidic skin treatment compositions. As will be described in greater detail hereinbelow, the oil moderates the undesirable effects of the acid, while preserving its beneficial effect. In many instances, delivery of the treatment composition may be a problem, particularly when the composition is formulated to include a relatively low viscosity carrier such as water. The use of cotton swabs, cotton wool and similar materials can result in either the over-application, under-application or uneven application of the treatment materials; and consequently, can either compromise the efficacy of the treatment program or result in waste and excess cost. Furthermore, open containers of the treatment composition are prone to spillage and contamination.

It would be desirable to have the skin treatment composition of the present invention disposed in a delivery system which provides for the simple and rapid dispensing of premeasured portions of the composition in a form which may be readily applied to the skin. The present invention provides an acidic based skin treatment composition which is non-irritating, non-allergenic, non-comedogenic and highly effective. The composition is packaged in premeasured portions which may be readily applied in a therapeutic setting. These and other advantages of the present invention will be readily apparent from the drawings, discussion and description which follow.

### Brief Description of the Invention

There is disclosed herein a composition for the topical treatment of skin, comprising by volume:
3% to 70% of an acidic material selected from the group consisting of:
glycolic acid, trichloroacetic acid and combinations thereof:
0.1-10% of lemon oil; and
the remainder, an inert carrier.

There is also disclosed herein a packaged composition for the topical treatment of skin comprising:
I) a body of bibulous material;
II) a liquid composition as described above, absorbed into said body of bibulous material;
III) a moisture-proof package configured to enclose and retain said body of bibulous material.

### Brief Description of the Figure

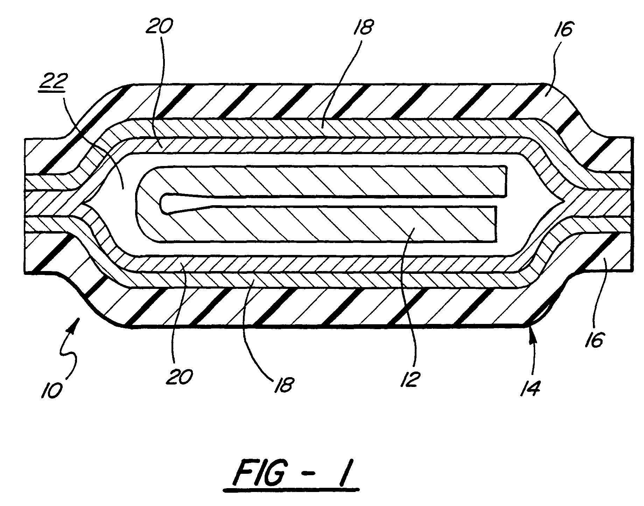

FIGURE 1 is a cross-sectional view of a package of skin treatment composition, structured in accord with the present invention.

### Detailed Description of the Invention

The invention is directed to a topical composition for treating a variety of skin conditions, including dry skin, dermatoses, acne, keratoses, photo-aging, melasma, itching, inflammation, pseudofolliculitis barbae (razor bumps) and the like. The composition includes an acidic component together with a limonene based oil and further includes a cosmetically acceptable carrier which may comprise water, a gel, a lotion or an ointment.

### The Acid

There are a variety of acidic materials which may be utilized to fabricate the compositions of the present invention, glycolic acid, trichloroacetic acid and combinations thereof.

In general, the acid component In general, the acid component comprises by volume 3-70% of the compositions of the present invention. The precise amount of acid employed will depend upon the strength of the acid as well as the manner in which the composition is employed. Relatively strong acids such as trichloroacetic acid will be used at lower concentrations as compared to milder acids. Acid concentration will also depend upon the manner in which the composition is used. Compositions which are intended to be dispensed by physicians or other trained professionals generally include relatively high acid concentrations since these materials will be applied therapeutically, in a controlled setting, and for restricted periods of time. Compositions for home use are generally of lower acid concentration.

### The Oil

In accord with the present invention it has been unexpectedly found that the inclusion of even relatively small amounts of lemon oil in an acidic skin treatment composition greatly improves the performance of the composition. The oil prevents stinging and burning associated with heretofore employed acidic compositions, while preserving the beneficial effects of the acid. It has also been found that the oil provides for the manufacture of a stable product having a smooth and uniform consistency, particularly when the compositions is disposed in an ointment or lotion based carrier.

The precise mode in which the oil functions is not fully understood at this time; however, the oil is a are complex natural product which is capable of reacting with various portions of the acid molecules to form new molecular structures. It is also possible that the oil may form a metastable complex with the acid. Alternatively, the oil may interact with the acid in a physical (i.e., non-chemical) manner as, for example, by solvating, emulsifying or sequestering the acid to limit its ionization, control, its solubility or otherwise moderate its effects. It has been found that approximately 0.1-10% of the oil, by volume, is sufficient to obtain the beneficial effects of the present invention. In one group of compositions of the present invention, the oil is present at a volume concentration of approximately 2-10%.

The beneficial effects obtained by the inclusion of the lemon oil into the acid-containing skin treatment compositions are quite surprising in view of the fact that prior art compositions often included carriers such as creams or lotions formulated from mineral oil, lanolin or other non-limonene oils. Therefore, the prior art would suggest that no additional benefit is to be obtained by inclusion of an oily material in acid based skin treatments.

### The Carrier

The composition of the present invention is compatible with any one of a number of generally employed and cosmetically acceptable carriers. The carrier material is inert in the sense that it does not interact with the active components of the composition. The carrier merely constitutes a vehicle for the delivery of the composition, although it is to be understood that the carrier itself may provide for some humidification or lubrication of the skin since it will typically include water, oil, glycerin and similar agents. The simplest carrier will merely comprise water and may further include coloring agents, scents. Aqueous carriers are generally favored for high concentration, therapeutic formulations which are typically applied in a professional setting for relatively short periods of time. Lower dosage compositions which are left on the skin for longer periods of time will typically include materials such as glycerin or gelling agents to retard evaporation. As is well known in the art, lotion and ointment based carriers typically comprise emulsions of oil and water stabilized with emulsifying agents and may further include thickeners, color and scent.

There are a large number of skin creams and lotions which are commercially available, and the composition of the present invention may incorporate such materials as the inert carrier. A typical commercially available skin cream comprises by weight approximately: 50% water, 10% mineral oil, 10% petrolatum, 10% of a fatty alcohol such as cetyl alcohol or stearyl alcohol, 5% propylene glycol, 5% isopropyl palmitate, 5% sodium lauryl sulfate together with preservatives, colorants and scents. The foregoing cream formulation may be mixed with water at a ratio of three parts water to one part cream to make a lotion. Many variations of this general formula are known and available in the art and may be employed in the practice of the present invention.

### The Package

As noted above, application of the composition can, in some instances, be a problem, particularly if the composition is of fairly low viscosity. It is generally desirable to apply the composition in a uniform layer and to minimize spilling or other wastage. In accord with the present invention, it has been found desirable to dispose acid based dermatologic compositions in a single use package. The package comprises a moisture-proof envelope, enclosing and retaining a body of bibulous material having the composition absorbed thereupon. The bibulous material typically comprises a woven or non-woven fabric, an absorbent cotton pad, a paper towel. The package seals and protects the composition until it is ready for use.

Referring now to Figure 1, there is shown a cross-sectional view of one particular embodiment of a packaged dermatologic composition 10 structured in accord with the principles of the present invention. The package 10 includes an absorbent member 12, which in this instance is a piece of absorbent cotton and rayon fabric, disposed within a water-proof package 14. The illustrated package 14 is comprised of an outer layer of paper 16 having a layer of aluminum foil 18 bonded thereto. The innermost layer is comprised of a layer of medium density polyethylene 20 and it will be noted that the package 10 is sealed along the edges by thermally bonding the polyethylene layers 20 so as to define an enclosed interior volume 22.

The Figure 1 package is representative of a variety of packages which may be employed in the present invention. It is to be understood that the package may, in some instances, be entirely made from a polymeric material or from a metal foil, or it may comprise paper impregnated with a polymer. The prime criterion is that the package be capable of retaining a pad saturated with the skin treatment composition, without leaking or reacting with the acidic material. While in the illustrated embodiment, the pad 12 is described as being a cotton and rayon fabric, other materials of a bibulous nature such as paper toweling, non-woven fabrics, gauze pads may be similarly employed. Also, the package of the present invention may be employed with non-limonene oil containing compositions of the type well-known in the art.

### Examples

A number of packaged formulations were prepared in accord with the present invention as follows:
1. A water based solution comprising 60% of glycolic acid was prepared as follows: 86 grams of glycolic acid (70% aqueous solution) were stirred together with 4 grams of lemon oil (food grade Durkee Co.) and 10 ml distilled water at room temperature. This provided a homogeneous mixture which was stable upon standing. The mixture comprised 60% glycolic acid, 4% lemon oil and 36% water. The foregoing composition was used to wet a number of pads made of a cotton-rayon fabric; each pad was 50.8mm (two inches) long by 76.2 mm (three inches) wide and contained approximately 0.0015 L (1.5cc) of the composition. The pads were each sealed in a polyethylene envelope which was enclosed in a labelled paper envelope.
2. A similar composition was prepared from 57 grams of the aforementioned glycolic acid solution, 4 grams of lemon oil and 39 ml of water. This mixture was also stable on standing and comprised 40% glycolic acid, 4% lemon oil and 56% water. The composition was applied to absorbent paper toweling pads approximately 50.8 mm (two inches) long by 76.2mm (three inches) wide. The pads were each wetted with 0.0015 L (1.5cc) of the composition and disposed in a package generally similar to that of Figure 1.
3. A third composition was prepared from 29 grams of the glycolic acid solution, 4 grams of lemon oil and 67 ml of water. This composition comprised 20% glycolic acid, 4% lemon oil and 76% water. This composition was applied to pads of cotton fabric approximately three inches square at a loading of 0.003 L (3cc) each. The pads were sealed in polyethylene bags.
4. A lotion based composition was prepared from the following components:
   isopropyl alcohol USP 7.68 L (7680 cc)
   propylene glycol 2.258 L (2258 cc)
   glycerin 0.3388 L (338.8 cc)
   laureth 4 (sodium laurel ethyl sulfate surfactant) 0.0678 L (67. 8cc)
   distilled water 0.0265 L (26.5 cc)
   glycolic acid, 70% aqueous solution 0.875 L (875 cc)
   oil of lemon 0.750 L (750cc)
   The lotion was prepared by stirring together the ingredients, in the order listed, at room temperature for approximately 1/2 hour until a homogeneous mixture was obtained. The composition was packaged in accord with Example 1 and was found to be particularly useful in the treatment of pseudofolliculitis barbae.
5. A comparative composition was prepared and packaged as per the preceding example except that the glycolic acid was replaced by a 70% aqueous solution of lactic acid, and oil of orange (food grade, Durkee Company) was substituted for the oil of lemon.

It is to be understood that the foregoing examples are merely illustrative of particular embodiments of the present invention and are not limitations upon the practice thereof. It is the following claims, including all equivalents, which define the scope of the invention.

## Claims

1. A composition for the topical treatment of skin, comprising by volume:
3% to 70% of an acidic material selected from the group consisting of:
glycolic acid, trichloroacetic acid and combinations thereof:
0.1-10% of lemon oil; and
the remainder, an inert carrier.

2. A composition as claimed in claim 1, wherein said inert carrier comprises water.

3. A composition as claimed in claim 1, wherein said inert carrier comprises an emulsion of oil and water.

4. A composition for the topical treatment of skin comprising by volume:
3-70% of glycolic acid;
0.1-10% of lemon oil; and the remainder, an inert carrier.

5. A packaged composition for the topical treatment of skin comprising:
I) a body of bibulous material;
II) a liquid composition as claimed in claim 4 absorbed into said body of bibulous material;
III) a moisture-proof package configured to enclose and retain said body of bibulous material.

6. A packaged composition as claimed in claim 5, wherein said bibulous material is selected from the group consisting of: woven textiles, non-woven textiles, paper and combinations thereof.

7. A packaged composition as claimed in claim 5, wherein said bibulous material is sealed in said moisture-proof package.

## Patentansprüche

1. Zusammensetzung für die topische Behandlung von Haut, umfassend, bezogen auf Volumen:
3% bis 70% eines sauren Materials, ausgewählt aus der Gruppe, bestehend aus:
Glycolsäure, Trichloressigsäure und Kombinationen davon,
0,1-10% Zitronenöl und
den Rest einen inerten Träger.

2. Zusammensetzung nach Anspruch 1, wobei der inerte Träger Wasser umfaßt.

3. Zusammensetzung nach Anspruch 1, wobei der inerte Träger eine Emulsion von Öl und Wasser umfaßt.

4. Zusammensetzung für die topische Behandlung von Haut, umfassend, bezogen auf Volumen:
3-70% Glycolsäure,
0,1-10% Zitronenöl und
den Rest einen inerten Träger.

5. Verpackte Zusammensetzung für die topische Behandlung von Haut, umfassend:
I) einen Körper von saugfähigem Material,
II) eine flüssige Zusammensetzung nach Anspruch 4, absorbiert in den Körper von saugfähigem Material,
(III) eine feuchtigkeitsundurchlässige Verpackung, konfiguriert, den Körper von saugfähigem Material zu umhüllen und beizubehalten.

6. Verpackte Zusammensetzung nach Anspruch 5, wobei das saugfähige Material ausgewählt ist aus der Gruppe, bestehend aus: Gewebe, Vliesstoff, Papier und Kombinationen davon.

7. Verpackte Zusammensetzung nach Anspruch 5, wobei das saugfähige Material in der feuchtigkeitsundurchlässigen Verpackung abgeschlossen ist.

## Revendications

1. Composition pour le traitement topique de la peau, comprenant en volume:
de 3% à 70% d'une matière acide choisie dans le groupe constitué:
d'acide glycolique, d'acide trichloroacétique et de combinaisons de ceux-ci;
0,1-10% d'essence de citron; et
le restant, un excipient inerte.

2. Composition suivant la revendication 1, dans laquelle ledit excipient inerte comprend de l'eau.

3. Composition suivant la revendication 1, dans laquelle ledit excipient inerte comprend une émulsion d'huile et d'eau.

4. Composition pour le traitement topique de la peau comprenant en volume:
3-70% d'acide glycolique;
0,1-10% d'essence de citron; et
le restant, un excipient inerte.

5. Composition emballée pour le traitement topique de la peau comprenant:
I) un corps de matière hydrophile;
II) une composition liquide suivant la revendication 4 absorbée dans ledit corps de matière hydrophile;
III) un emballage étanche à l'humidité configuré pour enfermer et assurer la conservation dudit corps de matière hydrophile.

6. Composition emballée suivant la revendication 5, dans laquelle ladite matière hydrophile est choisie dans le groupe constitué: de tissus tissés, de tissus non tissés, de papier et de combinaisons de ceux-ci.

7. Composition emballée suivant la revendication 5, dans laquelle ladite matière hydrophile est scellée dans ledit emballage étanche à l'humidité.
